# EUROPÄISCHE PATENTSCHRIFT

(11) **EP 1 253 622 B1**
(45) Veröffentlichungstag und Bekanntmachung des Hinweises auf die Patenterteilung: **12.12.2007**
(21) Anmeldenummer: 01250279.5
(22) Anmeldetag: 25.07.2001
(51) Int. Cl.: H01J 49/04, G06F 17/00, H01J 49/40

(54) **Verfahren zur Identifizierung von Mikroorganismen mittels MALDI-TOF-MS**
Method of identifying microorganisms using MALDI-TOF-MS
Méthode d'identification de microorganismes utilisant 'MALDI-TOF-MS'

(30) Priorität: 28.07.2000 DE 10038694
(43) Veröffentlichungstag der Anmeldung: 30.10.2002
(73) Patentinhaber: AnagnosTec Gesellschaft für Analytische Biochemie und Diagnostik mbH, 14476 Potsdam OT Golm (DE)
(72) Erfinder: Kallow, Wibke, Dr., 12279 Berlin (DE); Dieckmann, Ralf, Dr., 10707 Berlin (DE); Kleinkauf, Niels, Dr., 04229 Leipzig (DE); Erhard, Marcel, Dr., 14476 Potsdam, OT Golm (DE); Neuhof, Torsten, 14197 Berlin (DE)
(74) Vertreter: Schneider, Henry

(56) Entgegenhaltungen:
- US-A- 6 017 693

## Beschreibung

Die Erfindung betrifft ein Verfahren zur Identifizierung von Mikroorganismen mittels Matrix-assisted-Laser-Desorption-Ionisation Time-of-Flight Massenspektrometrie (MALDI-TOF-MS) mit den im Oberbegriff des Anspruchs 1 genannten Merkmalen sowie eine nach dem Verfahren erstellte und für das Verfahren verwendbare Datenbank.

Eine schnelle und zuverlässige Identifizierung von Mikroorganismen ist in verschiedenen Bereichen des Gesundheitswesens, beispielsweise der Diagnostik von Infektionen, sowie der Lebensmittelindustrie von entscheidender Bedeutung. Die traditionelle Identifizierung mittels des direkten Bakteriennachweises erfordert zunächst die Anzüchtung der zu identifizierenden Mikroorganismen aus einer Materialprobe. Anschließende mikroskopische Untersuchungsverfahren dienen hauptsächlich der vorläufigen Orientierung über den Bakteriengehalt sowie der Mikromorphologie beziehungsweise Färbeeigenschaften der klinischen Untersuchungsprobe. Die Identifizierung von Isolaten bis zur Speziesebene erfordert nicht selten eine Subkultivierung zwecks der Gewinnung einer Reinkultur. Nach dem klassischen Identifizierungsverfahren der medizinischen Mikrobiologie werden schließlich unter Verwendung einer geeigneten Kombination von Differentialmedien (so genannte "Bunte Reihe") spezifische Stoffwechselleistungen des zu identifizierenden Mikroorganismus erfasst. Hauptnachteil des mikrobiellen Verfahrens ist sein sehr hoher Zeitbedarf. Modernere molekularbiologische Ansätze, wie die PCR-Methode (Polymerase-Kettenreaktion) und die 16S-rRNA-Methode, involvieren die genetische Analyse des zuvor isolierten Genoms beziehungsweise bestimmter Ribonukleinsäuren. Diese Verfahren haben wegen ihrer hohen Empfindlichkeit stark an Bedeutung gewonnen. Sie erfordern ebenso wie die mikrobiologische Charakterisierung eine Kultivierung der Organismen im Labor und sind darüber hinaus mit einem erheblichen personellen und instrumentellen Aufwand belastet. Ferner ist ein infrarotspektroskopisches Verfahren entwickelt worden, bei dem Schwingungsspektren intakter Zellen ("Fingerprintspektren") in FTIR-Spektrometern aufgezeichnet und mit einer Datenbank mit Schwingungsspektren bekannter Mikroorganismen abgeglichen wird. Diese noch sehr neue Technik befindet sich noch in der Entwicklung und kann derzeit nur von speziell geschultem und erfahrenem Personal durchgeführt werden, so dass sich dieser Ansatz noch nicht in der Praxis durchsetzen konnte.

Aus US 6,017,693 ist ein Verfahren zur Identifizierung von Mikroorganismen bekannt, bei dem ein Proteinisolat des zu identifizierenden Mikroorganismus enzymatisch verdaut und anschließend einer fragmentierenden Tandem-Massenspektrometrie unterzogen wird. Die gemessenen Massenspektren werden dann mit Referenzmassenspektren verglichen, die anhand von Aminosäuresequenzen einer Proteindatenbank rechnerisch prognostiziert werden.

Mit der so genannten MALDI-TOF-Massenspektrometrie ist in den letzten Jahren ein Verfahren entwickelt worden, das im Gegensatz zu herkömmlichen massenspektrometrischen Verfahren auch der Analyse biologischer Makromoleküle zugänglich ist. Bei der MALDI-TOF-MS-Technik wird die zu untersuchende Probe mit einer meist kristallinen organischen Verbindung, der so genannten Matrix, auf eine Probenplatte gegeben, wobei die Probe in die Matrixkristalle eingebaut wird, und mit einem Laserstrahl in Wechselwirkung gebracht. Dabei werden einzelne Moleküle der Probe von dem Probenträger desorbiert und ionisiert. Anschließend werden die so erzeugten Ionen in einem elektrischen Feld beschleunigt und ihre Flugzeit (Time-of-Flight) bis zum Erreichen eines Detektors registriert. Da die Beschleunigung von der Masse eines ionisierten Moleküls abhängt, spiegeln die Flugzeiten die in der Probe vorhandenen Molekülmassen wider. Die MALDI-TOF-MS-Technik findet heutzutage hauptsächlich auf dem Gebiet der Proteinanalytik ("Proteomics") und bei der RNA- und DNA-Analytik Anwendung. Es wurde vorgeschlagen, die Methode zur Identifizierung von Mikroorganismen in Analogie zu der oben beschriebenen infrarotspektroskopischen Fingerprintmethode einzusetzen. Dafür wird das MALDI-TOF-Massenspektrum eines Zellextraktes oder intakter Zellen (zum Beispiel WO 98/09314) des unbekannten Mikroorganismus mit den Spektren bekannter Organismen verglichen. Der Vergleich des Probenspektrums mit den Referenzspektren der Datenbank erfolgt in der Regel computergestützt mittels statistisch-mathematischer Algorithmen, die in so genannten Mustererkennungsverfahren entwickelt wurden. Die Massenspektren ähneln sich mit wachsender verwandtschaftlicher Nähe der Mikroorganismen zunehmend. Das heißt, die Wahrscheinlichkeit einer Wiederholung bestimmter Signale in den Massenspektren nimmt zu. Daher ist das Verfahren bei übergeordneten Klassifizierungsniveaus, etwa bei Gattungen oder Familien, mit einer erhöhten Unsicherheit verbunden, wenn eine Zuordnung auf Stammniveau fehlschlägt, beispielsweise weil kein Referenzspektrum des Stammes in der Datenbank vorhanden ist. Das Verfahren ist daher auf sehr umfangreiche Datenbanken mit einer Vielzahl repräsentativer Referenzspektren von bekannten Stämmen angewiesen. Hinzu kommt die Schwierigkeit, ein spektrales Grundrauschen von echten Signalen zu diskriminieren.

Aufgabe der vorliegenden Erfindung ist es daher, das gattungsgemäße MALDI-TOF-MS-Verfahren zur Identifizierung von Mikroorganismen dahingehend weiterzuentwickeln, dass seine Zuverlässigkeit erhöht wird, ohne den erforderlichen Prozessaufwand zu vergrößern. Es soll ferner eine nach dem Verfahren erstellte und für das Verfahren einsetzbare Datenbank zur Verfügung gestellt werden.

Diese Aufgabe wird durch ein Verfahren mit den Merkmalen des Anspruchs 1 gelöst.

Verfahrensgemäß ist vorgesehen, dass als Referenzspektren synthetische Massenspektren verwendet werden, die durch Zusammenfassung einer gegenüber "natürlichen" (nicht reduzierten) Massenspektren reduzierten Anzahl von für den jeweiligen Mikroorganismus spezifischen Signalen aus den gemessenen Massenspektren der bekannten Mikroorganismen erzeugt werden. Durch die Reduzierung der Referenzspektren auf eine verhältnismäßig geringe Anzahl charakteristischer Signale, wird eine erhebliche Daten- und Informationsreduktion erreicht. Durch die Datenreduktion wird nicht nur Speicherplatz eingespart, sondern auch ein zeitlicher Aufwand für Datenübertragungen, so dass das Verfahren prinzipiell auch für eine Anwendung über vernetzte Datenverarbeitungsanlagen (z. B. Internet) geeignet ist. Darüber hinaus ermöglicht die Informationsreduktion der Referenzspektren der Datenbank eine deutliche Beschleunigung der Ähnlichkeitsanalyse des Massenspektrums des zu identifizierenden Mikroorganismus mit den Referenzspektren, da die Analyse sich nunmehr auf einen Vergleich der in den Referenzspektren enthaltenen Signale beschränken kann.

Verglichen mit herkömmlichen Verfahren, in denen ein Probenspektrum des zu identifizierenden Organismus mit "natürlichen" Referenzspektren abgeglichen wird, ist eine Zuverlässigkeit des erfindungsgemäßen Verfahrens, das heißt die Wahrscheinlichkeit, eine korrekte Zuordnung des unbekannten Organismus zu treffen, deutlich erhöht. Hierin ist der bedeutendeste Vorteil der Erfindung zu sehen. Die erhöhte Zuverlässigkeit ist durch die hohe Konzentrierung spezifischer Informationen in den Referenzspektren zu erklären, die nicht durch Signale geringer Signifikanz oder Rauschen überdeckt wird. Auch bei einem möglichen Versagen bei einer Zuordnung auf Stammebene einer Probe, etwa weil kein Referenzspektrum dieses Stammes in der Datenbank verfügbar ist, liefert das Verfahren zuverlässige Identifizierungen auf übergeordneten Klassifizierungsebenen, beispielsweise auf Gattungs- oder Artenebene. Die Empfindlichkeit der erfindungsgemäßen Vorgehensweise wird auch nicht durch Unterschiede in verschiedenen Spektren beeinträchtigt, welche beispielsweise durch unterschiedliche Kultivierungsbedingungen, unterschiedliche Zellstadien oder abweichende Signal-Rauschverhältnisse verursacht werden.

Ein weiterer Vorteil des Verfahrens stellt die geringe erforderliche Probenmenge des zu identifizierenden Mikroorganismus dar, die in kürzeren Kultivierungszeiten erhältlich ist. Ferner können auch Mischkulturen analysiert werden, so dass auf die Anzucht von Reinkulturen verzichtet werden kann.

Die in den synthetischen Referenzspektren enthaltenen Signale können in zwei Kategorien unterschieden werden. Eine besonders vorteilhafte Ausgestaltung der Erfindung sieht vor, dass die Signale eines Referenzspektrums mindestens ein identifiziertes Signal umfassen, welches einem charakterisierten molekularen Zellbestandteil des jeweiligen Mikroorganismus eindeutig zugeordnet wurde. Zur Identifizierung eines Mikroorganismus geeignete Zellbestandteile sind beispielsweise bestimmte Peptide, Proteine, Ribonukleinsäuren und/oder Lipide. Es hat sich als besonders vorteilhaft erwiesen, eine Signalzuordnung eines ribosomalen Proteins, insbesondere eines Proteins der großen Ribosomenuntereinheit, vorzunehmen. Für Bakterien sind dies Proteine der so genannten 50S-Untereinheit und bei Pilzen die 60S-Untereinheit. Proteine stellen einen Hauptbestandteil mikrobieller Zellen dar. Dies gilt insbesondere für Ribosomenproteine, die unabhängig von einem Entwicklungsstadium der Zelle, einem Nährstoffangebot oder sonstiger Kultivierungsbedingungen ständig präsent sind und somit zuverlässige Signale in den Massenspektren darstellen. Hinzu kommt, dass Aminosäuresequenzen analoger Proteine unterschiedlicher Spezies oder sogar unterschiedlicher Stämme sich zumindest geringfügig voneinander unterscheiden. Folglich weisen die analogen Proteine unterschiedliche Massen auf und eignen sich für deren Unterscheidung. So gelang den Erfindern beispielsweise erstmalig die Zuordnung von vier Massensignalen in dem Massenspektrum von *Escherichia coli* (m/z = 4365, 5381, 7276 und 5096) zu den Proteinen L36, L34 und L29 der großen 50S-Ribosomenuntereinheit beziehungsweise zu dem Protein S22 der kleinen 30S-Untereinheit. Diese Signale sind ständige Bestandteile in Massenspektren von *E. coli,* nicht jedoch vieler anderer Mikroorganismen. Sie sind daher besonders zur Identifizierung von *Escherichia coli* und zur Aufnahme in ein synthetisches Referenzspektrum für *E. coli* geeignet. Im Falle von Pilzen haben sich überdies die Strukturproteine Hydrophobine zur Identifizierung bewährt.

In einer weiteren Ausbildung der vorliegenden Erfindung ist vorgesehen, dass die Signale eines synthetischen Referenzspektrums als zweite Signalkategorie mindestens ein empirisches Signal umfassen, welches durch Vergleich einer Vielzahl von Massenspektren bekannter Mikroorganismen als spezifisch für einen Mikroorganismus ermittelt wurde. Dabei handelt es sich um Signale, deren Ursprung, das heißt deren verursachende molekulare Zellkomponente, zwar nicht bekannt ist, die jedoch aufgrund bestimmter Kriterien als charakteristisch für einen Mikroorganismus gewertet werden. Vorzugsweise umfassen diese Kriterien eine vorgebbare Mindest-Häufigkeit für das Auftreten eines Signals in einer Anzahl von Massenspektren des selben Mikroorganismus sowie eine vorgebbare durchschnittliche Mindest-Intensität des Signals. Dabei sollte die Mindest-Häufigkeit mindestens 50 %, insbesondere mindestens 70 %, vorzugsweise mindestens 90 %, betragen. Die Ermittlung der empirischen Signale durch Vergleich gemessener Massenspektren kann visuell, vorzugsweise jedoch computergestützt, durchgeführt werden. Entsprechende Algorithmen (Mustererkennungsverfahren) sind bekannt und sollen hier nicht näher erläutert werden.

Die Anzahl der Signale eines Referenzspektrums beträgt vorteilhafterweise 5 bis 30. In praktischen Versuchen hat sich insbesondere eine Anzahl von 10 bis 15 als ausreichend erwiesen. Es ist ferner bevorzugt vorgesehen, dass ein Signal eines synthetischen Referenzspektrums durch lediglich ein Koordinatenpaar repräsentiert wird. Dabei besteht das Koordinatenpaar aus einer Masse beziehungsweise einem Masse-Ladungs-Verhältnis als x-Koordinate einerseits und einer absoluten oder relativen Intensität als y-Koordinate andererseits. Verglichen mit "natürlichen" Massenspektren, die etwa 16000 Datenpunkte enthalten, bedeutet dies eine erhebliche Datenreduktion. Für den Abgleich eines Probespektrums mit den Referenzspektren der Datenbank können vorteilhafterweise den einzelnen identifizierten und empirischen Signalen der synthetischen Referenzspektren Gewichtungen entsprechend ihrer Signifikanz zugeordnet werden.

Die Erfindung umfasst ferner eine Datenbank, die synthetische Referenzspektren umfasst, welche aus gemessenen MALDI-TOF-Massenspektren einer Anzahl bekannter Mikroorganismen durch Zusammenfassung einer gegenüber natürlichen Massenspektren reduzierten Anzahl von für den jeweiligen Mikroorganismus spezifischen Signalen nach dem erfindungsgemäßen Verfahren erhalten wurde, sowie die Verwendung der Datenbank für die Identifizierung von Mikroorganismen mittels MALDI-TOF-MS.

Die Erfindung wird nachfolgend in Ausführungsbeispielen anhand der zugehörigen Zeichnungen näher erläutert. Es zeigen:
- Figur 1: ein Ablaufschema einer typischen Ausführung des erfindungsgemäßen Verfahrens;
- Figur 2: MALDI-TOF-Massenspektren von *Escherichia coli* und von isolierten Ribosomen aus *Escherichia coli;*
- Figuren 3 bis 8: synthetische Referenzspektren verschiedener Bakterien, insbesondere für zwei *Escherichia coli*-Stämme, zwei *Klebsiella*-Spezies, *Pseudomonas aeruginosa* und *Staphylococcus aureus;* und
- Figur 9: synthetisches Referenzspektrum des Pilzes *Trichoderma reesei.*

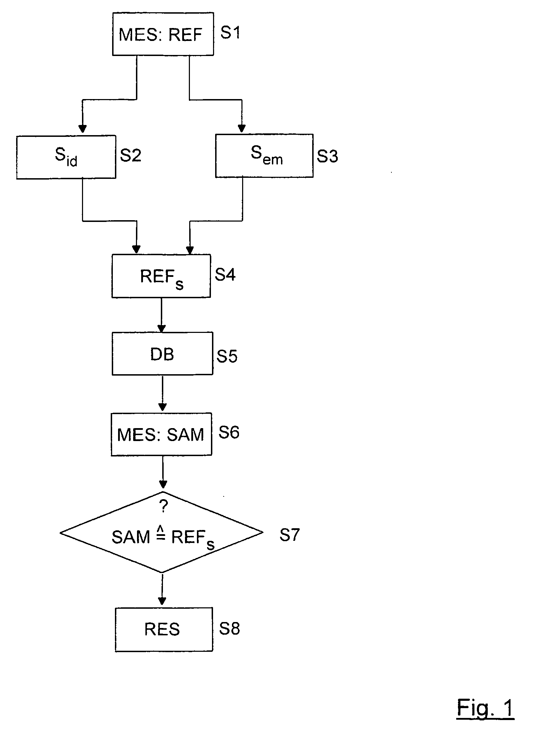

Figur 1 zeigt in einem Fließschema einen typischen erfindungsgemäßen Verfahrensablauf. In einem ersten Schritt S1 wird eine möglichst große Anzahl von Referenzspektren REF bekannter Mikroorganismen gemessen. In diesem Zusammenhang werden im Folgenden einige Details zur Probenvorbereitung und zur Datenakquisition gegeben.

### Probenvorbereitung und MALDI-TOF-Datenakquisition

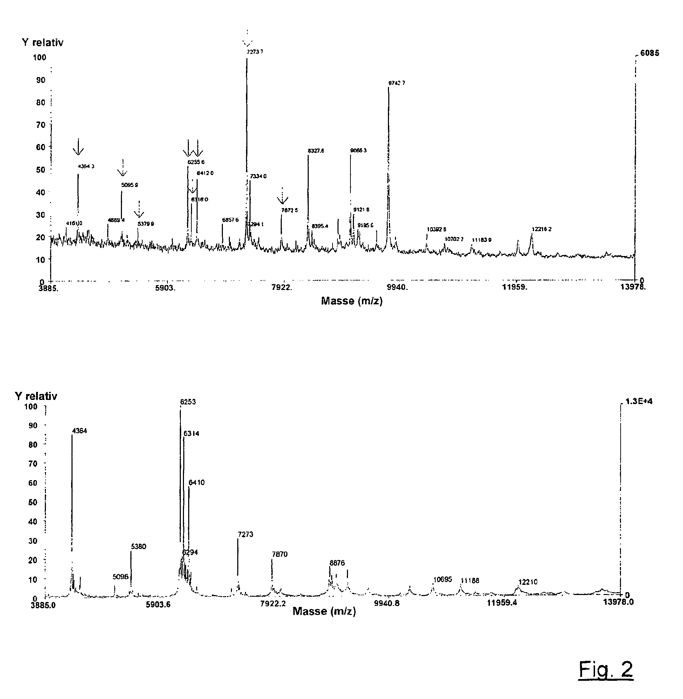

Für eine MALDI-TOF-Analyse werden zirka 5 bis 100 µg Nasszellen oder auch 5 bis 50 µg getrocknete Zellen eines bekannten oder zu identifizierenden Bakteriums oder Pilzes benötigt. Eine Vorbehandlung der Zellen, beispielsweise ein Zellaufschluss, ist nicht notwendig. Die Nasszellen können entweder direkt von einer AGAR-Kultur mit einer sterilen Impföse auf eine auch Template genannte Probenplatte übertragen werden. Alternativ können auch abzentrifugierte Zellen einer Flüssigkultur verwendet werden. Anschließend werden die Zellen auf der Probenplatte mit 0,2 bis 1 µl einer Matrixlösung vermischt. In Abwandlung von dieser Prozedur können die Nasszellen auch vor ihrer Übertragung auf die Probenplatte mit der Matrixlösung vermischt und als Suspension übertragen werden. Für die folgenden Messungen wurde eine Matrixlösung aus 100 mg/ml 2,5-Dihydroxybenzoesäure in einer Mischung aus 50 % Acetonitril und 50 % Wasser mit 3 % Trifluoressigsäure verwendet. Andere bekannte Matrixlösungen sind ebenfalls geeignet. Nach Trocknung der Probe, die mit einer Kristallbildung einhergeht, kann die Probe direkt einer MALDI-TOF-massenspektrometischen Analyse unterworfen werden. Für die vorliegenden Messungen wurde jeder Probenpunkt einer Probenplatte in einem herkömmlichen Massenspektrometer mit zirka 50 bis 300 Laserpulsen eines Stickstoff-Lasers mit einer Wellenlänge von 337 nm angeregt. Die Akquisition positiver Ionenmassenspektren erfolgte im Linearmessmodus in einem Massenbereich von 2000 bis 20000 m/z. Ein typisches Beispiel eines auf diese Weise erhaltenen positiven MALDI-TOF-Massenspektrums REF von *Escherichia coli* ist im oberen Teil der Figur 2 im Massenbereich von zirka m/z = 4000 bis 14000 dargestellt.

Im anschließenden Verfahrensschritt S2 (Figur 1) wird eine Peakzuordnung zu bestimmten molekularen Zellbestandteilen durchgeführt. Eine mögliche Vorgehens weise, die auf bekannte Methoden der Biochemie und Molekularbiologie zurückgreift, wird im Folgenden am Beispiel von Ribosomenproteinen der großen Ribosomenuntereinheit kurz erläutert.

### Identifizierung unbekannter Signale

Ein Proteinextrakt einer Zellkultur wird mittels einer 2D-Gelelektrophorese aufgetrennt. In Frage kommende so genannte Proteinspots werden anschließend einem tryptischen Verdau, bei dem das Protein enzymatisch in kleine Proteinbruchstücke gespaltet wird, unterzogen. Sofern Antikörper gegen Ribosomen vorhanden sind, können die relevanten Proteinspots auch durch ein Immunoassay (zum Beispiel Western-Blot-Analyse) erkannt werden. Die durch den tryptischen Verdau gewonnenen Proteinbruchstücke werden dann mittels einer HPLC, die für besonders kleine Probenmengen geeignet ist, aufgetrennt und ansequenziert. Mit den auf diese Weise ermittelten Sequenzbruchstücken kann dann versucht werden, die Ribosomengene in einer Datenbank zu identifizieren. Sind entsprechende Gene in der Datenbank vorhanden, kann aus der gesamten Gensequenz die korrespondierende Proteinmasse ermittelt werden. Kann ein entsprechendes Gen in der Datenbank nicht gefunden werden, so muss mit bekannten Mitteln der Molekularbiologie, die hier nicht näher ausgeführt werden sollen, das gesamte Gen, welches für das entsprechende ribosomale Protein kodiert, isoliert und sequenziert werden. Ist die Gensequenz bekannt, folgt die Übersetzung in die Proteinsequenz und Ermittlung der theoretischen Proteinmasse. Eine Überprüfung dieser theoretischen Masse kann erfolgen, indem der entsprechende Proteinspot der 2D-Gelelektrophorese einer MALDI-TOF-Massenspektrometrie unterworfen wird. Bei Abweichungen von der theoretischen Proteinmasse können Modifikationen des Proteins durch MALDI-TOF-Analyse des tryptischen Verdaus festgestellt werden.

Zur Verdeutlichung ist in der Figur 2 im unteren Teil ein Massenspektrum des 70S-Ribosomens aus *Escherichia coli,* das mittels 2D-Gelelektrophorese isoliert wurde, dargestellt. Mit 70S wird der gesamte Ribosomen eines Proteins bezeichnet, der sich aus der großen 50S-Untereinheit und der kleinen 30S-Untereinheit zusammensetzt. Beide Untereinheiten bestehen ihrerseits aus einer Reihe von Proteinen, die mit dem Buchstaben L (für large) und dem Buchstaben S (für small) bezeichnet werden. Es ist ohne weiteres ersichtlich, dass eine Reihe der intensivsten Signale aus dem Spektrum von *Escherichia coli* ATCC 25922(vgl. Figur 2 oberer Teil) sich auf ribosomale Proteine zurückführen lassen. Entsprechende Signale sind in der Abbildung mit Pfeilen gekennzeichnet. Den Erfindern ist es erstmalig gelungen, drei dieser Signale bestimmten Proteinen der großen 50S- und ein Signal einem Protein der kleinen 30S-Ribosomenuntereinheit zuzuordnen. Dabei handelt es sich um die Proteine L29, L34, L36 und S22 mit den Massen m/z = 7274, 5381, 4365 beziehungsweise 5096. Da insbesondere die Signale der großen Untereinheit mit einer hohen Zuverlässigkeit in den Massenspektren von *Escherichia coli* auftauchen, sind sie besonders zur Identifizierung geeignet. Sie wurden als identifizierte Signale S_{id} für das synthetische Referenzspektrum REFₛ für *Escherichia coli* verwendet.

In einem alternativen oder zusätzlichen Schritt S3 (Figur 1) werden empirische Signale Sₑₘ aus den gemessenen Referenzspektren REF der bekannten Mikroorganismen ermittelt. Die Ermittlung der empirischen Signale Sₑₘ erfolgt durch Vergleich einer Vielzahl von Massenspektren, die von demselben Stamm aufgezeichnet wurden, untereinander sowie durch Vergleich dieser Massenspektren mit denen von anderen Organismen. Dabei werden solche Signale als charakteristisch für einen Mikroorganismus ermittelt, die möglichst häufig in den Massenspektren desselben Organismus auftauchen und möglichst selten in den Massenspektren eines anderen. Für die Häufigkeit eines Auftretens eines geeigneten empirischen Signals Sₑₘ kann dabei ein Mindest-Wert, beispielsweise > 70 % bezogen auf alle Spektren eines Organismus, vorgegeben werden. Ebenso sollte ein solches Signal eine vorgebbare Mindest-Intensität besitzen, um seine Unterscheidung von dem Untergrundrauschen zu erleichtern. Die Ermittlung der empirischen Signale Sₑₘ im Schritt S3 kann durch visuellen Spektrenvergleich durch den Anwender erfolgen. Es ist jedoch bevorzugt vorgesehen, diesen Schritt automatisiert mit Hilfe geeigneter Rechnerprogramme durchzuführen. Hier kommt etwa ein Algorithmus in Frage, welcher die gemessenen Referenzspektren REF hinsichtlich der genannten Kriterien untersucht. Es sind jedoch auch, beispielsweise aus der Infrarotspektrometrie, computergestützte Verfahren bekannt, die unter Anwendung statistischer Algorithmen in der Lage sind, wiederkehrende Signale zu erkennen und herauszufiltern.

Die in den Schritten S2 und S3 ermittelten identifizierten und empirischen Signale S_{id}, Sₑₘ werden in einem anschließenden Schritt S4 zu synthetischen Referenzspektren REF_{S} zusammengefasst. Dabei wird für jeden bekannten Mikroorganismus ein Referenzspektrum REF_{S} erzeugt.

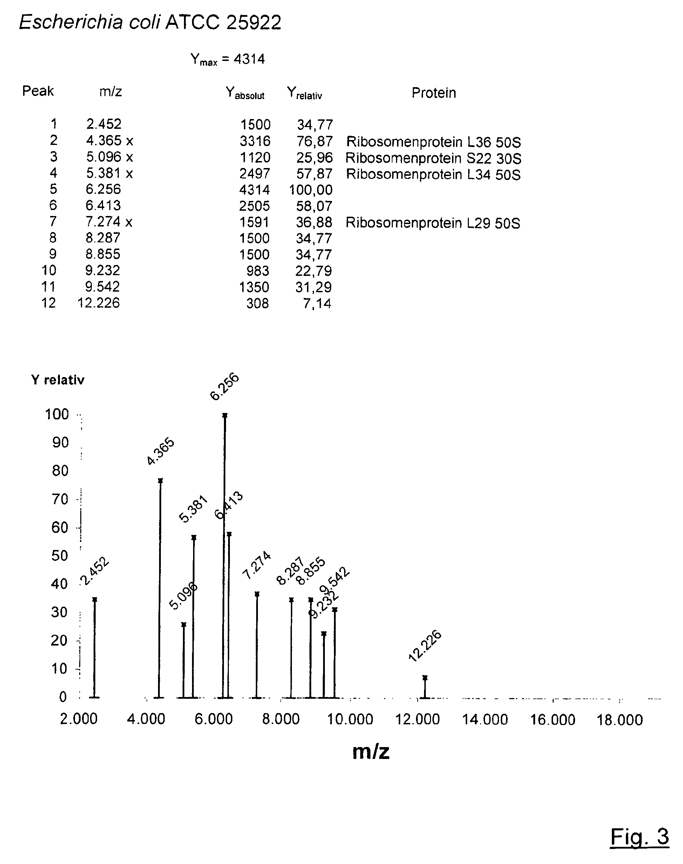

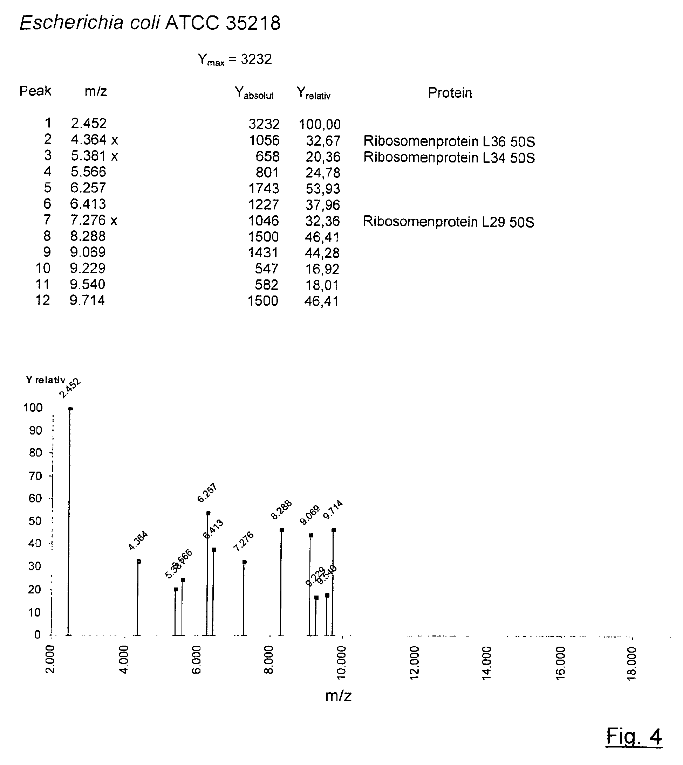

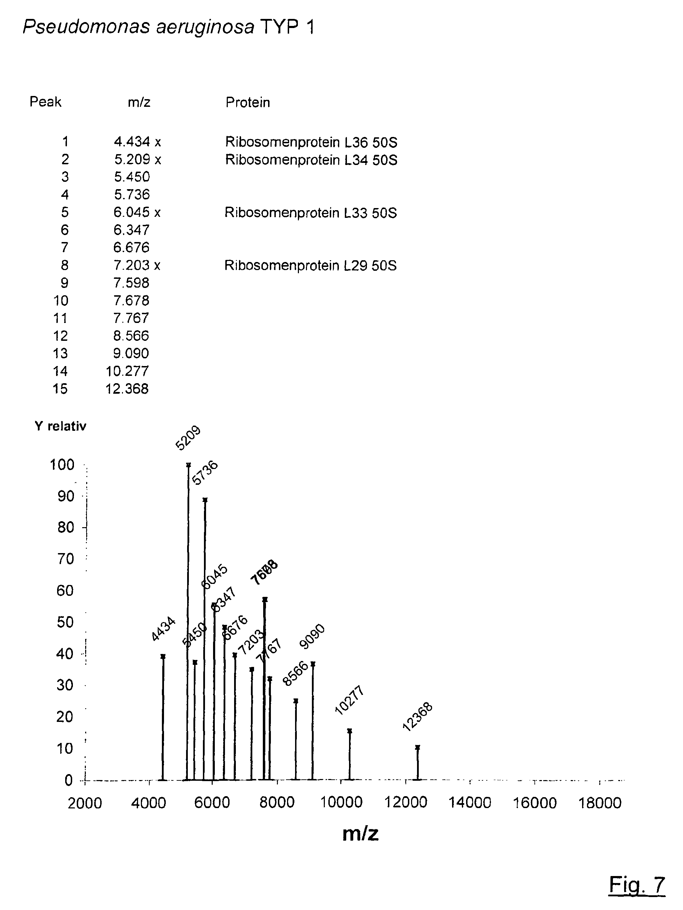

In den Figuren 3 bis 9 sind beispielhaft derartige synthetische Referenzspektren REF_{S} für zwei *Escherichia coli*-Stämme (ATCC 25922 und ATCC 35218), zwei *Klebsiella-*Spezies *-* nämlich *Klebsiella oxycoca* und *Klebsiella pneumoniae* -, für *Pseudomonas aeruginosa, Staphylococcus aureus* sowie für den Pilz *Trichoderma reesei* dargestellt. Dabei ist jeweils im oberen Teil jeder der Figuren 3 bis 9 das synthetische Referenzspektrum REF_{S} in Koordinatenform dargestellt (teilweise unter Verzicht der y-Koordinaten), während im unteren Teil jeweils die graphische Repräsentation in der typischen Form von Massenspektren abgebildet ist. Die synthetischen Referenzspektren REF_{S} umfassen in den gezeigten Beispielen zehn bis fünfzehn Signale S, im Falle des Pilzes *T. reesei* fünf. In den Referenzspektren REF_{S} von *Escherichia coli* (Figuren 3 und 4) und von *Pseudomonas aeruginosa* (Figur 7) sind die als Proteine der großen 50S-Ribosomenuntereinheit identifizierten Signale S_{id} markiert. Dabei konnten für *E. coli* und für *P. aeruginosa* jeweils die den Proteinen L29, L34 und L36 der großen Untereinheit zugehörigen Signale identifiziert werden sowie für *P. aeruginosa* zusätzlich das dem Protein L33 zugehörige Signal. Im Falle von *E. Coli* ATCC 25922 (Figur 3) konnte ferner ein Signal dem Protein S22 der kleinen 30S-Ribosomenuntereinheit zugeordnet werden.

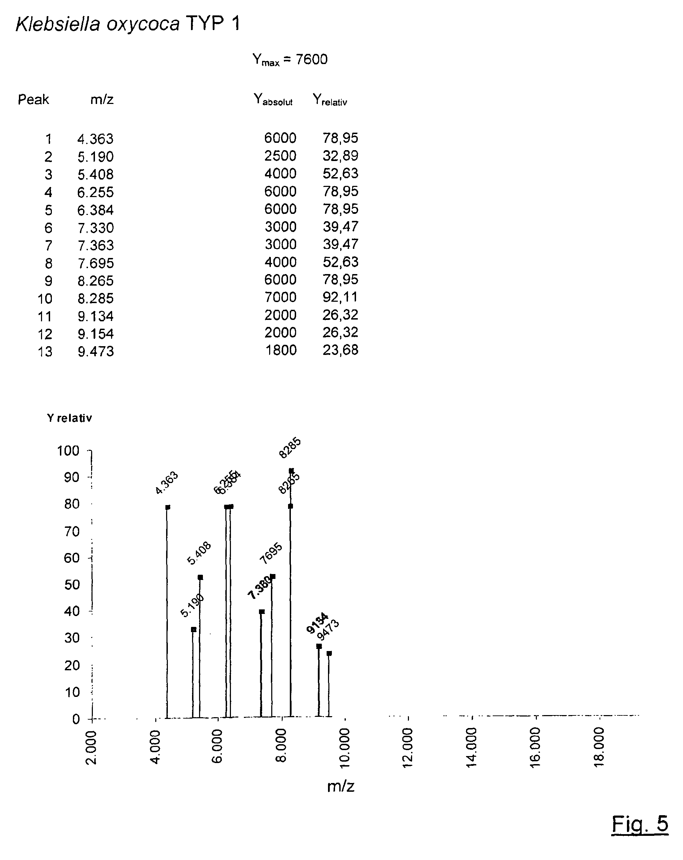

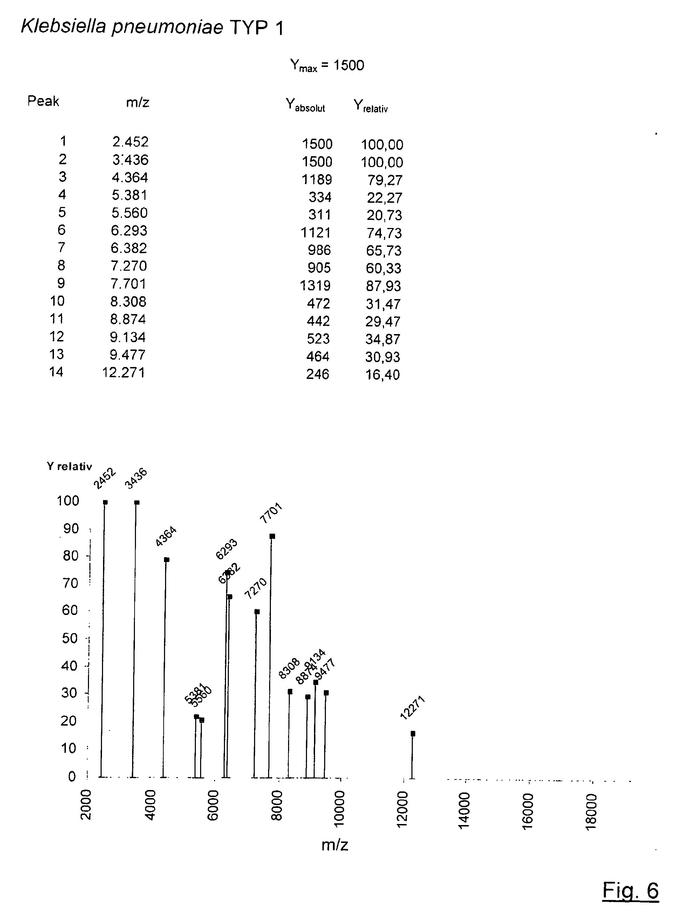

Die Unterscheidung der beiden *Escherichia coli*-Stämme untereinander (Figuren 3 und 4) wird hauptsächlich durch solche empirische Signale Sₑₘ ermöglicht, die nur bei einem von beiden Stämmen beobachtet werden. Ein Vergleich der in den Figuren 3 und 4 gezeigten synthetischen Referenzspektren REF_{S} zeigt, dass das erfindungsgemäße Verfahren empfindlich genug ist, um eine Unterscheidung von Mikroorganismen sogar auf Stammniveau zu ermöglichen. Daneben wird auch eine Unterscheidung zwischen *Escherichia coli* einerseits und *Klebsiella*-Spezies (Figuren 5 und 6) andererseits ermöglicht.

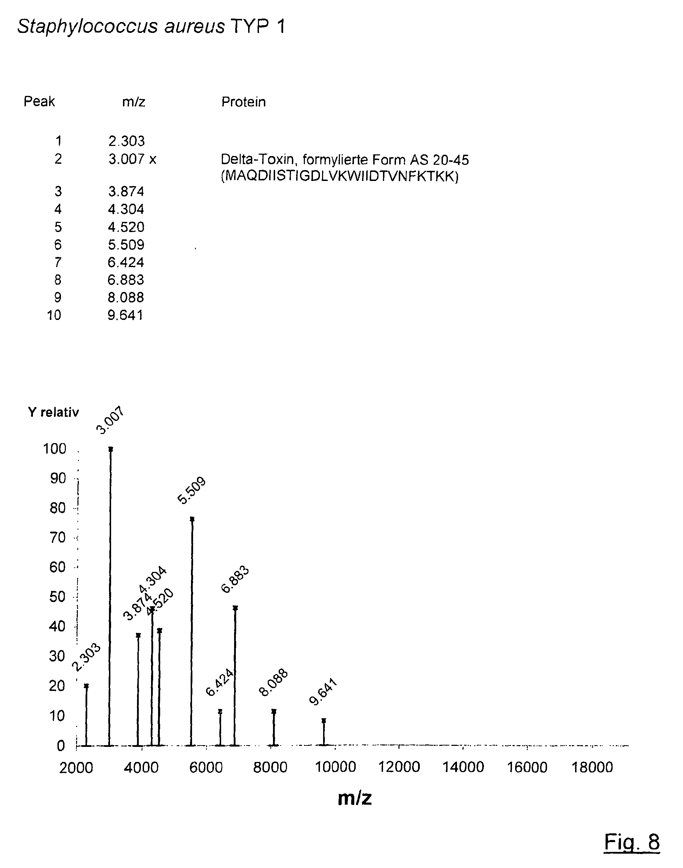

Das in Figur 8 dargestellte Beispiel von *Staphylococcus aureus* zeigt, dass auch andere als Ribosomenproteine identifiziert und zur Bestimmung des Organismus herangezogen werden können. In diesem Fall konnte ein Signal einem formylierten Peptidfragment des Delta-Toxins (Delta-Hemolysin) zugeordnet werden, das äußerst charakteristisch für dieses Bakterium ist.

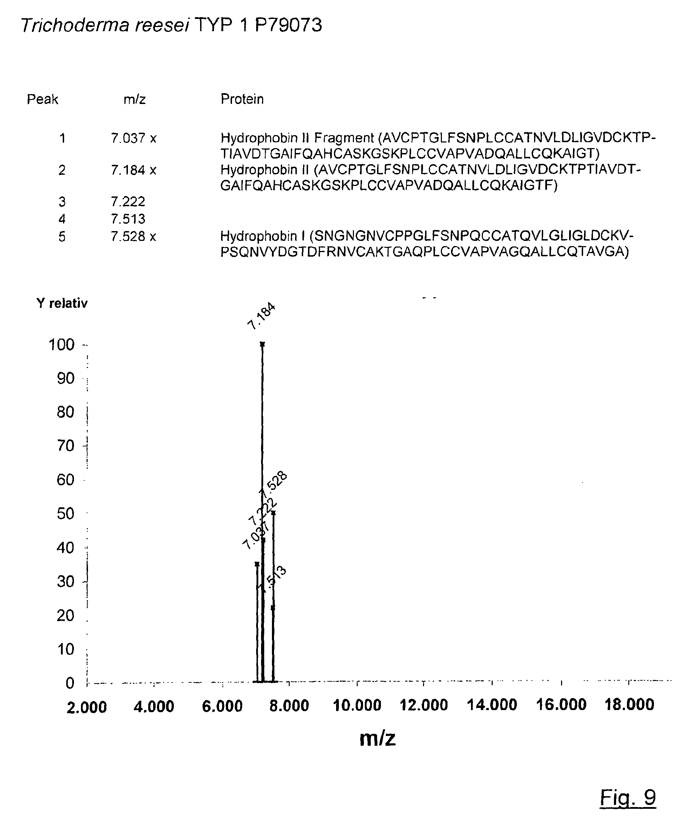

Im Falle von Pilzen haben sich neben ribosomalen Proteinen insbesondere auch die hydrophoben Strukturproteine Hydrophobine als vorteilhaft zur Identifizierung des Mikroorganismus erwiesen. So konnten für den Pilz *Trichoderma reesei* (Figur 9) drei Signale des Spektrums den Proteinen Hydrophobin I und II beziehungsweise einem Fragment von Hydrophobin II zugeordnet werden. Diese identifizierten Signale S_{id} können sowohl zur Abgrenzung gegenüber Bakterien, die keine Hydrophobine besitzen, als auch zur Differenzierung verschiedener Pilze untereinander herangezogen werden.

Die in dem Schritt S4 (Figur 1) erzeugten synthetischen Referenzspektren REF_{S} werden in einem anschließenden Schritt S5 zu einer Datenbank DB zusammengefasst. Innerhalb der Datenbank DB können die synthetischen Referenzspektren REF_{S} in logischer Weise, beispielsweise nach Familie, Gattung, Art und Stamm, geordnet werden. Der Verfahrensablauf von S1 bis S5, das heißt die Erstellung der Datenbank DB, muss prinzipiell nur einmal durchgeführt werden. Auf diese Datenbank DB kann dann in den anschließenden Schritten immer wieder zurückgegriffen werden. Selbstverständlich sollte die Datenbank DB ständig durch Referenzspektren REF_{S} weiterer Mikroorganismen erweitert und aktualisiert werden. So ist etwa denkbar, Referenzspektren REF_{S} von Mutanten, insbesondere solchen mit verändertem Resistenz- und/oder Virulenzverhalten, aufzunehmen, sofern diese Stämme charakteristische Signale liefern. Auch sollten auf Basis statistischer Auswertung der analysierten Proben die vorhandenen Referenzspektren REF_{S} laufend optimiert werden, beispielsweise durch die Aufnahme neuer Signale S_{id}, Sₑₘ und/oder durch Löschung von Signalen.

In dem Schritt S6 erfolgt dann die Akquisition eines Massenspektrums eines zu identifizierenden Mikroorganismus. Vorteilhafterweise wird dieses Probenspektrum SAM unter möglichst identischen Messbedingungen, insbesondere gleicher Matrix, wie die Referenzspektren REF in Schritt S1 gemessen. Nachfolgend (Schritt S7) erfolgt eine Ähnlichkeitsanalyse des Probenspektrums SAM mit den in der Datenbank DB enthaltenen synthetischen Referenzspektren REF_{S}. Auch dieser Spektrenabgleich kann visuell, vorzugsweise jedoch computergestützt, erfolgen. Dabei kann prinzipiell auf die gleichen oder ähnliche Algorithmen zurückgegriffen werden, die bereits in Schritt S3 zur Ermittlung der empirischen Signale Sₑₘ Anwendung fanden. Durch die Einfachheit der verwendeten Referenzspektren REF_{S} kann der Spektrenabgleich in Schritt S7 auch mit sehr einfachen Algorithmen durchgeführt werden, die sich lediglich auf einen Vergleich der Signale S_{id}, Sₑₘ der synthetischen Referenzspektren REF_{S} beschränken. Selbstverständlich müssen hier Toleranzkriterien vorgegeben werden, die festlegen, um wie viel ein in Frage kommendes Signal S_{id}, Sₑₘ des Probenspektrums SAM hinsichtlich der Masse und/oder der Intensität abweichen darf, um als übereinstimmend gewertet zu werden. Ferner sollte eine Mindest-Anzahl übereinstimmender Signale für eine Identifizierung vorgegeben werden. Im Schritt S8 erfolgt eine Ausgabe eines Resultats. Dieses kann entweder darin bestehen, dass eine Übereinstimmung des Probenspektrums SAM mit einem der Referenzspektren REF_{S} erkannt wurde. Ferner kann auch eine Information über den Grad der Übereinstimmung ausgegeben werden. Handelt es sich - wie häufig der Fall - bei der Probe des unbekannten Organismus um eine Mischkultur, so ermöglicht das Verfahren sogar die Identifizierung mehrerer, nebeneinander vorliegender Mikroorganismen. In einem solchen Fall werden als Ergebnis mehrere Treffer ausgegeben. Stimmt das Probenspektrum SAM mit keinem der synthetischen Referenzspektren REF_{S} innerhalb der zugelassenen Toleranzen überein, das heißt, der unbekannte Mikroorganismus ist nicht in der Datenbank DB enthalten, so ist auch dieses ein ausgebbares Resultat.

In bestimmten Fällen, in denen ein Mikroorganismus nicht oder nicht eindeutig identifiziert werden kann, kann vorteilhaft vorgesehen sein, dass im Anschluss an den Schritt S7 ein zusätzlicher Direktvergleich mit den gemessenen natürlichen" Referenzspektren REF durchgeführt wird. Dieses Vorgehen entspricht der bekannten Vorgehensweise zur Identifizierung von Mikroorganismen mittels MALDI-TOF-MS.

Durch die Einfachheit und den konzentrierten Informationsgehalt der synthetischen Referenzspektren REF_{S} zeichnet sich das erfindungsgemäße Verfahren durch eine wesentlich höhere Zuverlässigkeit aus.

### Bezugszeichenliste

- S1: Messung von Referenzspektren
- S2: Signalidentifizierung
- S3: Ermittlung empirischer Signale
- S4: Erzeugung eines synthetischen Referenzspektrums
- S5: Datenbankerstellung
- S6: Erfassung eines Probenspektrums
- S7: Spektrenabgleich
- S8: Ergebnisausgabe

- S_{id}: identifiziertes Signal
- Sₑₘ: empirisches Signal
- REF: gemessenes Referenzspektrum
- REF_{S}: synthetisches Referenzspektrum
- DB: Datenbank
- SAM: Probenspektrum
- RES: Ergebnis

## Patentansprüche

1. Verfahren zur Identifizierung von Mikroorganismen mittels Matrix-assisted-Laser-Desorption-Ionisation Time-of-Flight Massenspektrometrie, wobei
- eine Datenbank (DB) mit Referenzspektren (REF), welche aus gemessenen Massenspektren einer Anzahl bekannter Mikroorganismen erhalten werden, erstellt wird,
- ein MALDI-TOF-Massenspektrum (SAM) einer Probe eines zu identifizierenden Mikroorganismus aufgenommen wird,
- eine Ähnlichkeitsanalyse des Massenspektrums (SAM) des zu identifizierenden Mikroorganismus mit den in der Datenbank (DB) enthaltenen Referenzspektren (REF) durchgeführt wird,
**dadurch gekennzeichnet, dass** als Referenzspektren (REF) synthetische Massenspektren (REF_{S}) verwendet werden, die durch Zusammenfassung einer gegenüber natürlichen Massenspektren reduzierten Anzahl von für den jeweiligen Mikroorganismus spezifischen Signalen (S) aus den gemessenen Massenspektren der bekannten Mikroorganismen erzeugt werden.

2. Verfahren nach Anspruch 1, **dadurch gekennzeichnet, dass** die Signale (S) eines synthetischen Referenzspektrums (REF_{S}) mindestens ein identifiziertes Signal (S_{id}) umfassen, welches einem einzelnen molekularen Zellbestandteil des jeweiligen Mikroorganismus zugeordnet wurde.

3. Verfahren nach Anspruch 2, **dadurch gekennzeichnet, dass** der Zellbestandteil ein Peptid, ein Protein, eine Ribonukleinsäure und/oder ein Lipid ist.

4. Verfahren nach einem der vorhergehenden Ansprüche, **dadurch gekennzeichnet, dass** der Mikroorganismus ein Bakterium oder ein Pilz ist.

5. Verfahren nach einem der Ansprüche 3 oder 4, **dadurch gekennzeichnet, dass** der Zellbestandteil ein Protein, insbesondere ein ribosomales Protein ist.

6. Verfahren nach einem der Ansprüche 3 oder 4, **dadurch gekennzeichnet, dass** der Mikroorganismus ein Pilz und der Zellbestandteil ein Hydrophobin ist.

7. Verfahren nach einem der vorhergehenden Ansprüche, **dadurch gekennzeichnet, dass** die Signale (S) eines synthetischen Referenzspektrums (REF_{S}) mindestens ein empirisches Signal (Sₑₘ) umfassen, welches durch Vergleich von Massenspektren bekannter Mikroorganismen als spezifisch für einen Mikroorganismus ermittelt wurde.

8. Verfahren nach Anspruch 7, **dadurch gekennzeichnet, dass** der Vergleich visuell und/oder computergestützt durchgeführt wird.

9. Verfahren nach einem der Ansprüche 7 oder 8, **dadurch gekennzeichnet, dass** als Kriterien zur Ermittlung eines empirischen Signals (Sₑₘ) eine vorgebbare Mindest-Häufigkeit für ein Auftreten des Signals in einer Anzahl von Massenspektren des selben Mikroorganismus und eine vorgebbare Mindest-Intensität des Signals vorgegeben werden.

10. Verfahren nach einem der vorhergehenden Ansprüche, **dadurch gekennzeichnet, dass** ein Signal (S) durch ein Koordinatenpaar, bestehend aus einer Masse (m) oder einem Masse-Ladungsverhältnis (m/z) als x-Koordinate und einer absoluten oder relativen Intensität als y-Koordinate, repräsentiert wird.

11. Verfahren nach einem der vorhergehenden Ansprüche, **dadurch gekennzeichnet, dass** die Anzahl der Signale (S) eines synthetischen Referenzspektrums (REF_{S}) 5 bis 30, insbesondere 10 bis 15, beträgt.

12. Verfahren nach einem der vorhergehenden Ansprüche, **dadurch gekennzeichnet, dass** die Aufnahme der Massenspektren von nicht vorbehandelten Zellen durchgeführt wird.

13. Verfahren nach einem der vorhergehenden Ansprüche, **dadurch gekennzeichnet, dass** die Ähnlichkeitsanalyse des Massenspektrums (SAM) des zu identifizierenden Mikroorganismus mit den in der Datenbank (DB) enthaltenen synthetischen Referenzspektren (REF_{S}) sich auf einen Vergleich der in den synthetischen Referenzspektren (REFₛ) enthaltenen Signale (S) beschränkt.

14. Verfahren nach einem der vorhergehenden Ansprüche, **dadurch gekennzeichnet, dass** für die Ähnlichkeitsanalyse den in den synthetischen Referenzspektren (REF_{S}) enthaltenen Signalen (S) Gewichtungen zugeordnet werden.

## Claims

1. Method of identifying microorganisms using Matrix-assisted-Laser-Desorption-Ionisation Time-of-Flight Mass Spectrometry in which
- a data base (DB) is set up with reference spectra (REF) which are obtained from measured mass spectra of a number of known microorganisms,
- a MALDI-TOF-Mass Spectrum (SAM) of a sample of a microorganism which is to be identified is taken,
- a similarity analysis of the mass spectrum (SAM) of the microorganism which is to be identified is carried out with the reference spectra (REF) which are contained in the data base (DB),
**characterized in that** synthetic mass spectra (REFₛ) are used as reference spectra (REF) which are generated from the measured mass spectra of the known microorganisms by means of compiling a number of signals (S) specific for the respective microorganisms, the number being reduced when compared to natural mass spectra.

2. Method according to Claim 1 **characterized in that** the signals (S) of a synthetic reference spectrum (REFₛ) comprise at least one identified signal (S_{id}) which was assigned to an individual molecular cell component of the respective microorganism.

3. Method according to Claim 2 **characterized in that** the cell component is a peptide, a protein, a ribonucleic acid and/or a lipid.

4. Method according to any of the preceding claims **characterized in that** the microorganism is a bacterium or a fungus.

5. Method according to any of Claims 3 or 4 **characterized in that** the cell component is a protein, in particular a ribosomal protein.

6. Method according to any of Claims 3 or 4 **characterized in that** the microorganism is a fungus and the cell component is a hydrophobin.

7. Method according to any of the preceding claims **characterized in that** the signals (S) of a synthetic reference spectrum (REFₛ) comprise at least one empirical signal (Sₑₘ) which was determined as specific for a microorganism by means of the comparison of mass spectra of known microorganisms.

8. Method according to Claim 7 **characterized in that** the comparison is a visual and/or computer-aided comparison.

9. Method according to any of Claims 7 or 8 **characterized in that** a presettable minimum frequency for an occurrence of the signal in a number of mass spectra of the same microorganism and a presettable minimum intensity of the signal are preset as criteria for the determination of an empirical signal (Sₑₘ).

10. Method according to any of the preceding claims **characterized in that** a signal (S) is represented by a pair of coordinates consisting of a mass (m) or a mass-load ratio (m/z) as the x coordinate and an absolute or relative intensity as the y coordinate.

11. Method according to any of the preceding claims **characterized in that** the number of the signals (S) of a synthetic reference spectrum (REFₛ) is 5 to 30, in particular 10 to 15.

12. Method according to any of the preceding claims **characterized in that** the mass spectra are taken from cells which are not pre-treated.

13. Method according to any of the preceding claims **characterized in that** the similarity analysis of the mass spectrum (SAM) of the microorganism which is to be identified with the synthetic reference spectra (REFₛ) contained in the database (DB) is limited to a comparison of the signals (S) which are contained in the synthetic reference spectra (REFₛ).

14. Method according to any of the preceding claims **characterized in that** for the similarity analysis, weightings are assigned to the signals (S) which are contained in the synthetic reference spectra (REFₛ).

## Revendications

1. Méthode d'identification de micro-organismes au moyen de la spectrométrie de masse Matrix-assisted-Laser-Desorption-Ionisation Time-of-Flight,
- une banque de données (DB) étant établie, avec des spectres de référence (REF) qui sont obtenus à partir de spectres de masse mesurés d'un nombre de micro-organismes connus,
- un spectre de masse MALDI-TOF (SAM) d'un échantillon d'un micro-organisme à identifier étant enregistré,
- une analyse de similitude du spectre de masse (SAM) du micro-organisme à identifier avec les spectres de référence (REF) contenus dans la banque de données (DB) étant effectuée,
**caractérisée en ce que**, comme spectres de référence (REF), on utilise des spectres de masse synthétiques (REFₛ) qui sont produits par la réunion d'un nombre, réduit par rapport à des spectres de masse naturels, de signaux (S), spécifiques pour le micro-organisme respectif, issus des spectres de masse mesurés des micro-organismes connus.

2. Méthode selon la revendication 1, **caractérisée en ce que** les signaux (S) d'un spectre de référence synthétique (REFₛ) comprennent au moins un signal identifié (S_{id}) qui a été affecté à un composant cellulaire moléculaire individuel du micro-organisme respectif.

3. Méthode selon la revendication 2, **caractérisée en ce que** le composant cellulaire est un peptide, une protéine, un acide ribonucléique et/ou un lipide.

4. Méthode selon une des revendications précédentes, **caractérisée en ce que** le micro-organisme est une bactérie ou un champignon.

5. Méthode selon une des revendications 3 ou 4, **caractérisée en ce que** le composant cellulaire est une protéine, en particulier une protéine ribosomique.

6. Méthode selon une des revendications 3 ou 4, **caractérisée en ce que** le micro-organisme est un champignon et le composant cellulaire une hydrophobine.

7. Méthode selon une des revendications précédentes, **caractérisée en ce que** les signaux (S) d'un spectre de référence synthétique (REFₛ) comprennent au moins un signal empirique (Sₑₘ) qui a été établi par la comparaison des spectres de masse de micro-organismes connus en tant que spécifiques pour un micro-organisme.

8. Méthode selon la revendication 7, **caractérisée en ce que** la comparaison est effectuée visuellement et/ou de façon assistée par ordinateur.

9. Méthode selon une des revendications 7 ou 8, **caractérisée en ce que**, comme critères d'établissement d'un signal empirique (Sₑₘ), une fréquence minimale paramétrable de survenue du signal dans un nombre de spectres de masse du même micro-organisme et une intensité minimale paramétrable du signal sont paramétrées.

10. Méthode selon une des revendications précédentes, **caractérisée en ce qu'**un signal (S) est représenté par une paire de coordonnées, composée d'une masse (m) ou d'un rapport masse-charge (m/z) en tant que coordonnée x, et d'une intensité absolue ou relative en tant que coordonnée y.

11. Méthode selon une des revendications précédentes, **caractérisée en ce que** le nombre de signaux (S) d'un spectre de référence synthétique (REFₛ) est compris entre 5 et 30, en particulier entre 10 et 15.

12. Méthode selon une des revendications précédentes, **caractérisée en ce que** l'enregistrement des spectres de masse de cellules non prétraitées est effectué.

13. Méthode selon une des revendications précédentes, **caractérisée en ce que** l'analyse de similitude du spectre de masse (SAM) du micro-organisme à identifier avec les spectres de référence synthétiques (REFₛ) contenus dans la banque de données (DB) se limite à une comparaison des signaux (S) contenus dans les spectres de référence synthétiques (REFₛ).

14. Méthode selon une des revendications précédentes, **caractérisée en ce que**, pour l'analyse de similitude, des pondérations sont affectées aux signaux (S) contenus dans les spectres de référence synthétiques (REFₛ).
